# EUROPÄISCHE PATENTSCHRIFT

(11) **EP 3 544 928 B1**
(45) Veröffentlichungstag und Bekanntmachung des Hinweises auf die Patenterteilung: **06.01.2021**
(21) Anmeldenummer: 17787315.5
(22) Anmeldetag: 22.09.2017
(51) Int. Cl.: C01B 21/16, C07C 281/00, C25B 1/04, C25B 3/04, H01M 8/00, C25B 1/26, C25B 15/08, H01M 8/0606

(54) **VERFAHREN ZUR SPEICHERUNG VON ENERGIE IN FORM VON HYDRAZINCARBONAT**
METHOD FOR STORING ENERGY IN THE FORM OF HYDRAZINE CARBONATE
PROCÉDÉ POUR L'ACCUMULATION D'ÉNERGIE SOUS FORME DE CARBONATE D'HYDRAZINE

(30) Priorität: 22.11.2016 DE 102016223001
(43) Veröffentlichungstag der Anmeldung: 02.10.2019
(73) Patentinhaber: Schaeffler Technologies AG & Co. KG, 91074 Herzogenaurach (DE)
(72) Erfinder: HOLWEGER, Walter, 78736 Epfendorf (DE); WEGENER, Moritz, 91058 Erlangen (DE); MUSAYEV, Yashar, 90425 Nürnberg (DE)
(86) Internationale Anmeldenummer: PCT/DE2017/100808
(87) Internationale Veröffentlichungsnummer: WO 2018/095458

(56) Entgegenhaltungen:
- AT-A1- 514 461
- AT-A1- 516 196
- DE-A1- 3 030 324
- US-A- 3 077 383

## Beschreibung

Die Erfindung betrifft ein Verfahren zur Speicherung von Energie in Form von Hydrazincarbonat und zur anschließenden Rückumwandlung des Hydrazincarbonats in verwertbare Energie.

Aus der AT 514 461 A1 geht ein Verfahren zur Speicherung von Energie in Form von Hydrazincarbonat und anschließende Rückumwandlung des Hydrazincarbonats in verwertbare Energie hervor. Mit Hilfe von Energie und unter Zuführung von Stickstoff und Kohlendioxid wird unter Anwesenheit eines Übergangsmetallkatalysators, der die Aktivierungsenergie der stabilen Stickstoff-Stickstoff Dreifachbindung vermindert, in einem Elektrolyseverfahren in Wasser Hydrazincarbonat hergestellt. Die Anode ist dabei mit einem sauerstoffundurchlässigen Diaphragma umgeben. Ein Metallsalz wird als Inhibitor einer hydrogenolytischen Spaltung des Hydrazins eingesetzt. Das Hydrazincarbonat wird in Form eines Feststoffs oder in Form einer konzentrierten Lösung aus der Mischung isoliert, wobei das Hydrazincarbonat später thermisch zersetzt wird, um die Energie durch Verbrennung des Wasserstoffs oder durch Verwendung des Wasserstoffs in einer Brennstoffzelle wieder zu gewinnen.

Die US 3 077 383 A beschreibt ein Verfahren zur Herstellung von Hydrazinhydrat. Dabei werden Natriumhypochlorit und Ammoniak in Gegenwart einer Carbonylkomponente umgesetzt.

Die Aufgabe der vorliegenden Erfindung besteht darin, ein kostengünstiges und großtechnisch verwendbares Verfahren zur dauerhaften Speicherung von Energie in Form von Hydrazincarbonat sowie die anschließende Rückumwandlung des Hydrazincarbonats in verwertbare Energie vorzuschlagen. Insbesondere besteht die Aufgabe der vorliegenden Erfindung darin, eine nachhaltige Energiespeicherung ausgehend von gut verfügbaren Rohstoffen zu schaffen.

Nach dem erfindungsgemäßen Verfahren zur Speicherung von Energie in Form von Hydrazincarbonat und die anschließende Rückumwandlung des Hydrazincarbonats in verwertbare Energie, wird zunächst Meerwasser in einem Elektrolyseverfahren zur Herstellung von Hypochlorit verwendet, wobei das Hypochlorit durch Einleitung von Ammoniak über die Bildung von Monochloramin zu Hydrazin reagiert, und wobei anschließend das Hydrazin durch Einleitung von Kohlenstoffdioxid zu Hydrazincarbonat reagiert, wobei das Hydrazincarbonat zur Freigabe der gespeicherten Energie durch Reaktion an einem edelmetallfreien Katalysator Wasserstoff oder zumindest ein Wasserstoff enthaltendes Gas freigibt, und wobei der Wasserstoff oder das zumindest Wasserstoff enthaltende Gas zur Abgabe der verwertbaren Energie dazu vorgesehen ist, einer Brennstoffzelle zugeführt oder verbrannt zu werden. Insbesondere ist das Wasserstoff enthaltende Gas ein Gasgemisch aus zumindest Wasserstoff und Stickstoff.

Sowohl das Hydrazin als auch das Hydrazincarbonat sind aus dem allgemein bekannten Stand der Technik als auch aus der eingangs genannten AT 514 461 A1 bekannt. Demnach weist Hydrazin eine wässrige Form auf und wird großtechnisch vor allem als Reduktionsmittel und als Radikalfänger verwendet. Hydrazincarbonat ist ein nichtflüchtiges, in Wasser lösliches Salz, das in der chemischen Industrie als Antioxidans verwendet wird.

Das Meerwasser (H₂O) wird durch Meerwasserelektrolyse zur Herstellung von Hypochlorit (OCl⁻) verwendet. Dabei finden die nachfolgenden Reaktionen statt:

| | | | |
|---|---|---|---|
| Kathoden reaktion: | 2H₂O + 2e⁻ | → | H₂ + 2OH⁻ |
| Anodenreaktion: | 2Cl⁻ - 2e⁻ | → | Cl₂, |
| Reaktion 1: | Cl₂ + 2OH | → | H₂O + OCl⁻ + Cl⁻ |

Die in der Meerwasserelektrolyse zur Herstellung von Hypochlorit verwendete Energie wird vorzugsweise aus erneuerbaren Energiequellen gewonnen. Insbesondere setzt sich diese Energie aus Windkraftenergie, Sonnenenergie und/oder Bioenergie zusammen.

Das Hypochlorit reagiert durch Einleitung von Ammoniak (NH₃) über die Bildung von Monochloramin (NH₂Cl) zu Hydrazin (N₂H₄). Mit anderen Worten wird zunächst gasförmiges Ammoniak in die Hypochlorit umfassende wässrige Lösung eingeleitet, wobei gemäß Reaktion 2 neben Wasser auch Monochloramin gebildet wird.

| | | | |
|---|---|---|---|
| Reaktion 2: | NH₃ + HOCl | → | NH₂Cl + H₂O |

Gemäß einer darauffolgenden Reaktion 3 reagiert das Monochloramin mit dem Ammoniak und bildet neben Wasserstoffchlorid (HCl) auch Hydrazin.

| | | | |
|---|---|---|---|
| Reaktion 3: | NH₂Cl + NH₃ | → | N₂H₄ + HCl |

Anschließend reagiert gemäß Reaktion 4 das Hydrazin durch die Einleitung von Kohlenstoffdioxid (CO₂) zu Hydrazincarbonat ((N₂H₅)₂CO₃).

| | | | |
|---|---|---|---|
| Reaktion 4: | N₂H₄+ CO₂ | → | (N₂H₅)₂CO₃ |

Vorzugsweise wird das Kohlenstoffdioxid aus einem Abgasstrom einer Verbrennungsanlage entnommen. Beispielsweise wird der Abgasstrom durch ein Kohlekraftwerk erzeugt. Besonders vorteilhaft ist die Ausnutzung der hohen Konzentration von Kohlenstoffdioxid im Abgasstrom der Verbrennungsanlage.

Das Hydrazincarbonat dient als stabiler, dauerhafter Speicher für Wasserstoff (H₂). Zur Freigabe der im Hydrazincarbonat gespeicherten Energie wird ein edelmetallfreier Katalysator verwendet, wobei gemäß Reaktion 5 der Wasserstoff oder zumindest das Wasserstoff enthaltende Gas freigegeben wird.

Vorzugsweise wird als edelmetallfreier Katalysator ein Katalysator aus oxidischen Werkstoffen, wie beispielsweise aus Al₂O₃, Cu-dotiertem Al₂O₃ oder einem Calcium-Aluminium-Silikat wie CaAl₂Si₃O₈(OH)₄·H₂O, verwendet. An einem solchen edelmetallfreien Katalysator, wie beispielsweise CaAl₂Si₃O₈(OH)₄·H₂O, wird das Hydrazincarbonat, insbesondere bei Temperaturen im Bereich von 100 bis 150 °C, in Wasserstoff, CO₂ und Wasser zersetzt. Insbesondere weist der Katalysator eine hohe Porosität auf. Die Porosität dient insbesondere dazu, die reaktive Oberfläche des Katalysators zu vergrößern.

| | | | |
|---|---|---|---|
| Reaktion 5: | (N₂H₅)₂CO₃ | → | N₂+H₂+ CO₂+ H₂O |

Bevorzugt wird nach der Reaktion des Hydrazincarbonats an dem edelmetallfreien Katalysator ein Separationsschritt zur Anreicherung des Wasserstoffs vorgesehen. Insbesondere ist der Separationsschritt dazu vorgesehen, den Wasserstoff vom Stickstoff zu trennen. Bei dem gemäß Reaktion 5 freigegebenen Kohlenstoffdioxid handelt es sich um das zuvor eingebrachte und in dem Hydrazincarbonat reversibel gebundene Kohlenstoffdioxid. Mithin ist das erfindungsgemäße Verfahren CO₂-neutral.

Das Hydrazincarbonat wird zur Freigabe der gespeicherten Energie, insbesondere zur Freigabe des Wasserstoffs oder zur Freigabe des zumindest das Wasserstoff enthaltende Gas durch Reaktion an dem edelmetallfreien Katalysator auf weniger als 100°C erwärmt. Bevorzugt wird der Wasserstoff oder zumindest das Wasserstoff enthaltende Gas einer Brennstoffzelle zugeführt oder verbrannt, um die verwertbare Energie Abzugeben. Beispielsweise wird der Wasserstoff oder zumindest das Wasserstoff enthaltende Gas einer Niedertemperaturbrennstoffzelle zugeführt.

Die Nutzung des Wasserstoffs in Brennstoffzellen wirkt insbesondere der Diskontinuität der Stromversorgung durch erneuerbare Energiequellen entgegen. Ferner kann der im Hydrazincarbonat gespeicherte Wasserstoff sowohl für Brennstoffzellen in stationären Anwendungen als auch für Brennstoffzellen in mobilen Anwendungen wie PKW, LKW, Schiffen und Flugzeugen genutzt werden.

## Patentansprüche

1. Verfahren zur Speicherung von Energie in Form von Hydrazincarbonat und anschließende Rückumwandlung des Hydrazincarbonats in verwertbare Energie, **dadurch gekennzeichnet, dass** zunächst Meerwasser in einem Elektrolyseverfahren zur Herstellung von Hypochlorit verwendet wird, wobei das Hypochlorit durch Einleitung von Ammoniak über die Bildung von Monochloramin zu Hydrazin reagiert, und wobei anschließend das Hydrazin durch Einleitung von Kohlenstoffdioxid zu Hydrazincarbonat reagiert, wobei das Hydrazincarbonat zur Freigabe der gespeicherten Energie durch Reaktion an einem edelmetallfreien Katalysator Wasserstoff oder zumindest ein Wasserstoff enthaltendes Gas freigibt, und wobei der Wasserstoff oder das zumindest Wasserstoff enthaltende Gas zur Abgabe der verwertbaren Energie dazu vorgesehen ist, einer Brennstoffzelle zugeführt oder verbrannt zu werden.

2. Verfahren nach Anspruch 1,
**dadurch gekennzeichnet, dass** nach der Reaktion des Hydrazincarbonats an dem edelmetallfreien Katalysator ein Separationsschritt zur Anreicherung des Wasserstoffs vorgesehen wird.

3. Verfahren nach einem der vorhergehenden Ansprüche,
**dadurch gekennzeichnet, dass** der Wasserstoff oder zumindest das Wasserstoff enthaltende Gas einer Niedertemperaturbrennstoffzelle zugeführt wird.

4. Verfahren nach einem der vorhergehenden Ansprüche,
**dadurch gekennzeichnet, dass** das Kohlenstoffdioxid aus einem Abgasstrom einer Verbrennungsanlage entnommen wird.

5. Verfahren nach einem der vorhergehenden Ansprüche,
**dadurch gekennzeichnet, dass** als edelmetallfreier Katalysator ein Katalysator aus oxidischen Werkstoffen verwendet wird.

6. Verfahren nach einem der vorhergehenden Ansprüche,
**dadurch gekennzeichnet, dass** die in der Meerwasserelektrolyse zur Herstellung von Hypochlorit verwendete Energie aus erneuerbaren Energiequellen gewonnen wird.

7. Verfahren nach einem der vorhergehenden Ansprüche,
**dadurch gekennzeichnet, dass** das Hydrazincarbonat zur Freigabe der gespeicherten Energie durch Reaktion an dem edelmetallfreien Katalysator auf weniger als 100°C erwärmt wird.

## Claims

1. A method for storing energy in the form of hydrazine carbonate and subsequent reconversion of the hydrazine carbonate into reusable energy, **characterised in that** sea water is first used in an electrolysis method to produce hypochlorite, the hypochlorite reacting to form hydrazine by the introduction of ammonia, by means of the formation monochloramine, and subsequently the hydrazine reacts to form hydrazine carbonate by the introduction of carbon dioxide, the hydrazine carbonate releasing hydrogen or at least one hydrogencontaining gas by reaction on a catalyst free of noble metal, in order to release the stored energy, and the hydrogen or the gas containing at least hydrogen is to be supplied to a fuel cell or burned in order to emit the reusable energy.

2. The method according to claim 1,
**characterised in that** a separation step for enriching the hydrogen is provided after the reaction of the hydrazine carbonate on the catalyst free of noble metal.

3. The method according to any one of the preceding claims,
**characterised in that** the hydrogen or at least the gas containing the hydrogen is fed to a lowtemperature fuel cell.

4. The method according to any one of the preceding claims,
**characterised in that** the carbon dioxide is taken from an exhaust gas stream of a combustion plant.

5. The method according to any one of the preceding claims,
**characterised in that** a catalyst composed of oxidic materials is used as the catalyst free of noble metal.

6. The method according to any one of the preceding claims,
**characterised in that** the energy used in the sea water electrolysis for producing hypochlorite is obtained from renewable energy sources.

7. The method according to any one of the preceding claims,
**characterised in that** the hydrazine carbonate is heated to less than 100°C in order to release the stored energy by reaction on the catalyst free of noble metal.

## Revendications

1. Procédé d'accumulation d'énergie sous la forme de carbonate d'hydrazine et de reconversion ultérieure du carbonate d'hydrazine en énergie utilisable, **caractérisé en ce que** l'eau de mer est d'abord utilisée dans un procédé d'électrolyse destiné à la fabrication d'hypochlorite, dans lequel l'hypochlorite réagit, par introduction de l'ammoniac, à l'hydrazine par formation de monochloramine, et dans lequel l'hydrazine réagit ensuite, par introduction du dioxyde de carbone, au carbonate d'hydrazine, dans lequel le carbonate d'hydrazine libère, pour libérer l'énergie accumulée, par réaction sur un catalyseur sans métal noble, l'hydrogène ou au moins un gaz contenant de l'hydrogène, et dans lequel l'hydrogène ou le gaz contenant au moins de l'hydrogène, pour fournir l'énergie utilisable, est amené à une pile à combustible ou brûlé.

2. Procédé selon la revendication 1,
**caractérisé en ce que,** après la réaction du carbonate d'hydrazine sur le catalyseur sans métal noble, une étape permet la séparation pour enrichir l'hydrogène.

3. Procédé selon l'une quelconque des revendications précédentes,
**caractérisé en ce que** l'hydrogène ou au moins le gaz contenant de l'hydrogène est amené à une pile à combustible à basse température.

4. Procédé selon l'une quelconque des revendications précédentes,
**caractérisé en ce que** le dioxyde de carbone est prélevé dans un écoulement de gaz d'échappement d'une installation de combustion.

5. Procédé selon l'une quelconque des revendications précédentes,
**caractérisé en ce qu'**un catalyseur de matières oxydiques est utilisé en tant que catalyseur sans métal noble.

6. Procédé selon l'une quelconque des revendications précédentes,
**caractérisé en ce que** l'énergie utilisée lors de l'électrolyse de l'eau de mer pour fabriquer l'hypochlorite est produite à partir de sources d'énergie renouvelables.

7. Procédé selon l'une quelconque des revendications précédentes,
**caractérisé en ce que** le carbonate d'hydrazine, pour libérer l'énergie accumulée, est chauffé par réaction sur le catalyseur sans métal noble à moins de 100 °C.
